# EUROPÄISCHE PATENTSCHRIFT

(11) **EP 1 562 559 B1**
(45) Veröffentlichungstag und Bekanntmachung des Hinweises auf die Patenterteilung: **20.06.2012**
(21) Anmeldenummer: 03785518.6
(22) Anmeldetag: 14.11.2003
(51) Int. Cl.: A61K 9/51, A61K 47/48, C08G 63/06

(54) **BIOABBAUBARE KOLLOIDALE PARTIKEL, INSBESONDERE FÜR PULMONALE APPLIKATIONEN**
BIO-DEGRADABLE COLLOID PARTICLES, IN PARTICULAR FOR PULMONARY APPLICATIONS
PARTICULES COLLOIDALES BIODEGRADABLES NOTAMMENT DESTINEES A DES APPLICATIONS PULMONAIRES

(30) Priorität: 15.11.2002 DE 10253623
(43) Veröffentlichungstag der Anmeldung: 17.08.2005
(73) Patentinhaber: Justus-Liebig-Universität Gießen, 35390 Giessen (DE); Philipps-Universität Marburg, 35032 Marburg (DE)
(72) Erfinder: SEEGER, Werner, 35444 Biebertal (DE); SCHMEHL, Thomas, 35390 Giessen (DE); GESSLER, Tobias, 35435 Wettenberg (DE); DAILEY, Lea, Ann, San Francisco, CA 94121 (US); WITTMAR, Matthias, 59964 Medebach (DE)
(74) Vertreter: Buchhold, Jürgen
(86) Internationale Anmeldenummer: PCT/DE2003/003787
(87) Internationale Veröffentlichungsnummer: WO 2004/046202

(56) Entgegenhaltungen:
- EP-A- 1 132 416
- DE-A- 19 839 515
- US-A1- 2001 047 074
- KISSEL T ET AL.: "Protein delivery systems based on branched biodegradable polyesters" PROCEED. INT'L. SYMP. CONTROL. REL. BIOACT. MATER., Bd. 29, 2002, Seiten 286-287, XP008030039 ISSN: 1022-0178
- BEHRENS I ET AL.: "Self-assembling insulin-polymer complexes for peroral delivery: Interaction with Caco-2 cell monolayers, peptide transport and cytotoxicity" PROCEED. INT'L. SYMP. CONTROL. REL. BIOACT. MATER., Bd. 29, 2002, Seiten 881-882, XP008030038 ISSN: 1022-0178
- Wittmar M und Kissel T, The synthesis of polyesters with amine-modified poly(vinyl alcohol) backbones: A novel class of positively charged biopolymers for drug delivery and DNA vaccination, Europolymer Congress, Eindhoven, 2001

## Beschreibung

Die Erfindung betrifft eine Zusammensetzung aus einem biologisch abbaubaren amphiphilen Kammpolymer basierend auf einem Polyol-Rückgrat mit positiv geladenen Seitenketten und einem Zusatzstoff, insbesondere einem sauren Arzneimittelwirkstoff, zur Behandlung von Erkrankungen beim Menschen und höheren Säugetieren.

Der Erfolg von Behandlungen mit pharmazeutischen Wirkstoffen hängt in starkem Maße von deren Bioverfügbarkeit im Organismus ab. Die Bioverfügbarkeit hängt wiederum stark mit der jeweiligen Applikationsform des Wirkstoffs zusammen. In den letzten Jahren wurden deshalb neue Verabreichungstechniken mit dem Ziel entwickelt, die Anwendung möglichst einfach und patientenschonend zu gestalten. In einer Vielzahl von Fällen wird dabei auf nanopartikuläre Transportvehikel vertraut, die die Wirkstoffe aufnehmen und am gewünschten Ort freisetzen können. Als allgemein vorteilhaft gelten dabei solche partikularen Systeme deren Träger bioabbaubar sind.

Ein wichtiger Applikationsweg für Medikamente und Wirkstoffe zur Behandlung von Erkrankungen der Lunge ist die inhalative Verabreichung. Die inhalative Applikation von Medikamenten wird darüber hinaus zunehmend zur Behandlung von systemische Erkrankungen eingesetzt, da eine Vielzahl von Wirkstoffen, die alveolär deponiert werden, schnell in das Blut übertreten. Für beide Anwendungen ist ein nanopartikuläres Transportvehikel vorteilhaft, weil die Wirkstoffe nach Deposition der Nanopartikel in der Lunge kontinuierlich freigesetzt werden, so dass konstante Wirkstoffspiegel erzielt werden und sich die Anzahl der erforderlichen Inhalationen reduzieren lässt. Eine wichtige Voraussetzung für die inhalative Anwendung eines nanopartikulären Transportvehikels ist jedoch die Zerstäubungsstabilität, d.h. die Stabilität der Vehikel gegenüber dem Prozess der Aerosolerzeugung.

In Breitenbach, Kissel Polymer 39, Seite 3261, (1998) wird z.B. ein Verfahren zur Herstellung von bioabbaubaren Kammpolymeren, die als Trägerkomponenten für solche Partikel dienen können, beschrieben. Die Synthese der Polymere erfolgt ausgehend von einem Polyvinylakohol-Rückgrat an das hydrophobe Poly(milch-co-glycolsäure) Seitenketten (PVA-g-PLG) gepfropft werden.

In Weiterführung der Arbeiten mit diesen Kammpolymeren wird die Einführung von Sulfobutyl-Seitengruppen in das PVA-g-PLG-Kammpolymer in DE19839515 und EP1132416 beschrieben, wobei die erhaltenen Polymere als Träger für peptidische Wirkstoffe, insbesondere für Impfstoffe genutzt werden. Demgegenüber gelang es bisher nicht geeignete Polymere auf Basis solcher Polyol-Kammpolymere zur Verfügung zustellen, die für den Transport negativ geladener Wirkstoffe geeignet sind. In Kissel et al. (2002) Proceed. Int'l. Symp. Control. Rel. Bioact. Mater. 29, 286-287 ist die Herstellung eines nanopartikulären Trägers aus DEAPA-PVAL-g-PLGA zur Komplexierung von Insulin beschrieben. Der Träger dient dem Transport des Insulins und dessen Schutz beim Transport durch den Gastrointestinaltrakt. Behrens et al. (2002) Proceed. Int'l. Symp. Control. Rel. Bioact. Mater. 29, 881-882 beschreibt nanopartikuläre Komplexe aus DEAPA-PVAL-g-PLGA und Insulin und deren Interaktion mit Caco-2 Zellen. US 2001/0047074 offenbart Komplexe von Polyester aus Polyvinylalkohol mit PLGA oder negativ geladenen Poly(2-sulfobutyl)-Gruppen und deren Adhäsion an Caco-2 Zellen, die insbesondere zur oralen Applikation geeignet sind.

In Kissel et al. (2002) Proceed. Int'l. Symp. Control. Rel. Bioact. Mater. 29, 286-287 ist die Herstellung eines nanopartikulären Trägers aus DEAPA-PVAL-g-PLGA zur Komplexierung von Insulin beschrieben. Der Träger dient dem Transport des Insulins und dessen Schutz beim Transport durch den Gastrointestinaltrakt.

Behrens et al. (2002) Proceed. Int'l. Symp. Contyrol. Rel. Bioact. Mater. 29, 881-882 beschreibt nanopartikuläre Komplexe aus DEAPA-PVAL-g-PLGA und Insulin und deren Interaktion mit Caco-2 Zellen.

US 2001/0047074 offenbart Komplexe von Polyester aus Polyvinylalkohol mit PLGA oder negativ geladenen Poly(2-sulfobutyl)-Gruppen und deren Adhäsion an Caco-2 Zellen, die insbesondere zur oralen Applikation geeignet sind.

Somit ist die Bereitstellung eines partikularen Transportsystem insbesondere für negativ geladene Arzneimittelwirkstoffe, bevorzugt zur inhalativen Applikation, wünschenswert. Dabei ist es vorteilhaft, wenn das partikularen Transportsystem zerstäubungsstabil ist.

Die Aufgabe wird durch bioabbaubare kolloidale Partikel gelöst, wie sie in anspruch 1 angegeben sind.

Solche Kammpolymere, ob sie nun mit positiv geladenen Seitenketten (quartäre Aminogruppen) oder in ungeladener Form vorliegen bilden alleine keine stabilen kolloidalen Partikel, insbesondere nicht in wässrigen Lösungen. In Gegenwart organischer Lösemittel die mit Wasser mischbar sind, wie sie bei der Herstellung kolloidaler Partikel verwendet werden, sowie nach der Entfernung des organischen Lösemittels, bleiben die Polymere häufig in gelöster Form erhalten und zeigen die gewünschte Partikelbildung nicht. Somit können mit den beschriebenen aminogruppenhaltigen Kammpolymeren alleine keine definierbaren, stabilen kolloidalen Partikel erzeugt werden.

Es konnte allerdings beobachtet werden, dass unter Verwendung der oben beschriebenen Kammpolymere sehr stabile kolloidale Partikel hergestellt werden können, wenn negativ geladene Verbindungen, insbesondere Polyanionen, wie z.B. Dextran-Sulfat, Carboxymethylcellulose oder Nukleinsäuren bei der Herstellung der Partikel zugesetzt werden. Aber auch die Stabilisierung der Partikel mit kleineren negativ geladenen Molekülen ist möglich. Dabei werden insbesondere durch einen Überschuss von negativ geladenen Stabilisatoren sehr stabile Partikel in einem Größenbereich von 50 nm bis 1 µm erhalten, wobei die Partikelgröße von dem Stabilisator, aber auch von dem Überschuss an negativen Ladungen im Partikel abhängt, wodurch auch leicht Partikel mit einem Durchmesser von deutlich unter 1 µm erhalten werden können. Damit eröffnet sich der Weg die Größe der Partikel auf ein gewünschtes Maß einzustellen. Dabei sind für pharmazeutische Applikationen in der Regel kleinere Partikel von 50 nm bis 500 nm, insbesondere von 100 bis 250 nm, bevorzugt, die genügend Wirkstoff aufnehmen können und gleichzeitig, besonders im Hinblick einer inhalativen Aufnahme des Wirkstoffes, eine feine Verteilung zulassen. Dabei sind die erhaltenen kolloidalen Partikel sehr stabil, was insbesondere im Hinblick auf die geringe Neigung der reinen Kammpolymere Kolloide zu bilden überrascht.

Der Über- oder Unterschuss an negativen Ladungen der nötig ist, um stabile kolloidale Partikel zu generieren, kann durch die Bestimmung des Zeta-Potentials der Partikel bestimmt werden. Dabei sind in Abhängigkeit des verwendeten Stabilisators, Zeta-Potentiale zwischen -5 und -80 mV oder zwischen +5 und +80 mV geeignet. Bevorzugt sind Zeta-Potentiale zwischen -10 und -50 mV oder zwischen +10 und +50 mV. Dabei liegt das Zeta-Potential insbesondere für Polyanionen, niedermolekulare negativ geladenen Ionen und Mischungen aus Polyanionen und niedermolekularen Wirkstoffen besonders bevorzugt zwischen - 20 und -40 mV und zwischen +20 und +40 mV.

Insbesondere geeignete Wirkstoffe sind saure pharmazeutische Wirkstoffe. Besonders bevorzugt lassen sich pharmazeutische Wirkstoffe ausgewählt aus der Gruppe der Carbonsäuren, Sulfonsäuren oder Phosphorsäuren zu kolloidalen Partikeln verarbeiten, wobei über eine Säure-Base Reaktion die Aminogruppen des Polymers quartärnisiert und der Wirkstoff in ein negativ geladenes Molekül umgewandelt wird.

Ein Beispiel für einen solche Wirkstoffgruppe stellen die Prostanoide dar, worunter z. B. Iloprost ®, ein Wirkstoff zur Behandlung von pulmonaler Hypertonie fällt.

Wirkstoffe deren Azidität nicht ausreichend ist oder die direkt als negativ geladenes Molekül bei der Herstellung der kolloidalen Partikel verwendet werden, können z. B. in Begleitung einer Säure verarbeitet werden. Auch der Zusatz weiterer Hilfsstoffe, wie z.B. von Tensiden, bei der Herstellung von kolloidalen Partikeln ist möglich, wenn auch nicht nötig.

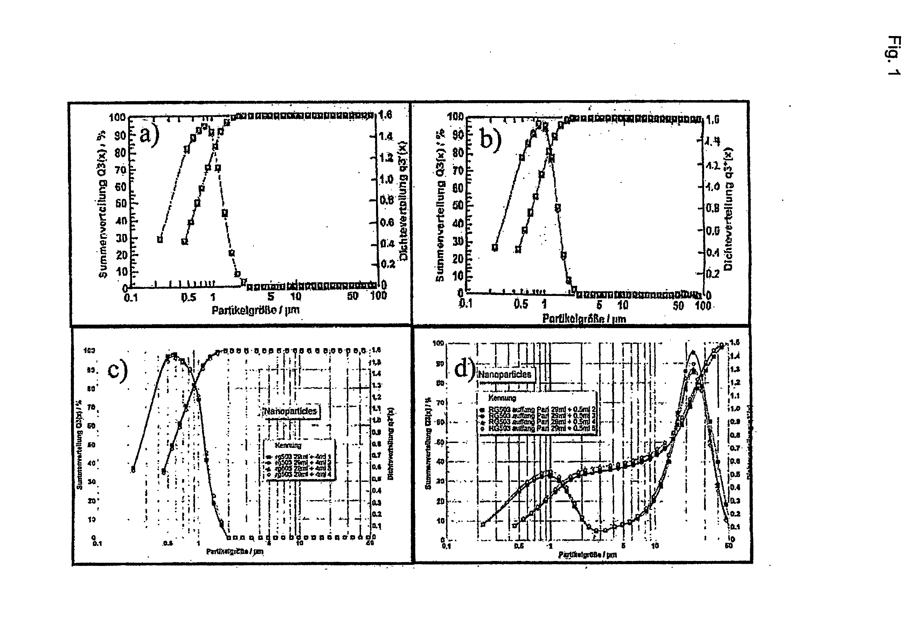

Insbesondere eignen sich die beanspruchten kolloidalen Partikel zur inhalativen Applikation von pharmazeutischen Wirkstoffen, da sie sich aus der wässrigen Phase unverändert zerstäuben lassen, wie dies z. B. Fig. 1 zeigt. Dazu können handelsübliche Zerstäuber wie z. B. Ultraschall-Vernebeler, piezoelektrische Vernebeler oder Jet-Vernebeler verwendet werden. Als geeignete Vernebeler lassen sich z. B. der Pari LC Star (Jet-Vernebeler; Pari Werk GmbH, Starnberg, Deutschland), der Pulmosonic (Ultraschall-Vernebeler; DeVilbiss, Langen, Deutschland) oder der Omron U1 (piezo-elektrischer Vernebeler; Omron Healthcare GmbH, Hamburg, Deutschland) einsetzen.

Um eine Aggregation der kolloidalen Partikel in solchen Vernebelern zu verhindern müssen die Transportpartikel in besonderem Maße stabil gegenüber Scherkräften, Ultraschall oder mechanischen Schwingungen sein, da sonst die praktische Anwendbarkeit von kolloidalen Suspensionen für inhalative Applikationen stark beeinträchtigt wird.

Weiterhin kann gezeigt werden, dass der Partikeldurchmesser problemlos in einem Bereich von unter 1 µm nach der Zerstäubung gehalten werden kann, also in einem Durchmesserbereich der für eine Deposition in der Lunge vorteilhaft ist. Dabei scheint überraschend die Zerstäubungsstabilität der kolloidalen Partikel mit abnehmender Hydrophobizität des Kammpolymers zuzunehmen.

Im Gegensatz dazu bilden viele herkömmlichen Kolloide bei der Zerstäubung einen hohen Anteil an Partikelaggregaten mit einem Durchmesser von über 5 µm, wodurch eine effiziente Verteilung des Aerosols in der Lunge nicht mehr möglich ist.

Durch die Zerstäubungsstabilität von kolloidalen Partikeln mit einem Durchmesser von kleiner 1 µm wird darüber hinaus die Aufnahme einer hohen Wirkstoffmenge durch Einatmen und die feine Wirkstoffverteilung der eingeatmeten Partikel in der Lunge gewährleistet. Somit kann die Bioverfügbarkeit eines inhalierten Wirkstoffes durch die Verwendung der erfindungsgemäßen Partikeln stark erhöht werden. Aus den genannten Gründen erschließt sich die einfache und patientenschonende inhalative Wirkstoffapplikation über die alveolare Region der Lunge durch die Verwendung der beanspruchten kolloidalen Partikel.

Darüber hinaus werden die Partikel biologisch langsam abgebaut, so dass eine kontinuierliche Wirkstofffreisetzung über längere Zeiträume gewährleistet ist. Somit eignen sich die beanspruchten Partikel in besonderem Maße als Transportvehikel zur Behandlung von pulmonalen und systemischen Erkrankungen sowohl beim Menschen als auch bei höheren Tieren.

Insbesondere die positiv geladenen kolloidalen Partikel eignen sich zur mucosalen Applikation von pharmazeutischen Wirkstoffen, da sie besonders gut an Schleimhäuten haften bleiben und durch adsorptive Endocytose aufgenommen werden können. Die Verwendbarkeit der Partikel ist bleibt aber nicht auf die genannten Applikationen beschränkt, sondern es können auch andere Verabreichungsformen, wie z.B. die sublinguale, buccale, orale, nasale, vaginale, okulare oder gastrointestinale Applikation, gewählt werden.

Die erfindungsgemäßen kolloidalen Partikel können z.B. nach folgendem Verfahren gewonnen werden:
a) lösen eines Kammpolymers, enthaltend ein wasserlösliches Polyol-Rückgrat, hydrophobe Seitenketten und sekundäre, tertiäre oder quartäre Aminogruppen tragende Seitenketten in Aceton,
b) versetzen einer isotonischen wässrigen Lösung mit einem pH-Wert von 7,0 enthaltend neben einem Zucker, namentlich Glucose und einem Puffer, namentlich Tris- EDTA, CMC, mit der aus a) erhaltenen Lösung,
c) rühren der aus (b) erhaltenen Lösung zur Herstellung kolloidaler Partikel und
d) entfernen des Acetons.

Zur Herstellung der Kammpolymere kann ein Polyolrückgrat, wie in Breitenbach, Kissel Polymer 39, Seite 3261, (1998) oder in EP1132416 beschrieben, mit hydrophoben Seitenketten gepfropft werden. Die Hydrophobizität der resultierenden Kammpolymere kann schon beim Propfen (z. B. mittels Melt-Grafting-Verfahren) durch die Menge an zugesetzten Seitenkettenmonomeren eingestellt werden. Für Polyvinylalkohole mit ca. 300 Hydroxygruppen hat sich der Zusatz von Lactiden/Glycoliden in einem Gewichtsverhätnis von 1:1 bis 1:50, bevorzugt von 1:5 bis 1:30 bewährt.

Die Anbindung der Aminogruppen tragenden Seitenketten erfolgt in der Regel vor der Pfropfung mit den hydrophoben Seitenketten.

Im folgenden werden die Erfindung an einigen Ausführungsbeispielen verdeutlicht, die nicht als einschränkend anzusehen sind.

### Beispiel 1: Herstellung von Polyvinylakohol-Kammpolymeren mit Aminogruppen tragenden Seitenketten

### 1.1. Herstellung von tertiären Carbonylimidazolalkylaminen

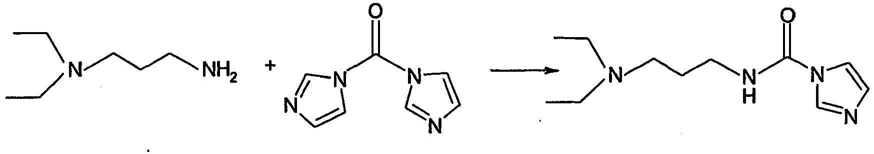

1,62 g (10 mmol) Carbonyldiimidazol (CDI) werden in 20 ml wasserfreiem THF gelöst, anschließend werden 10 mmol eines Amins (3-Dimethylaminopropylamin, 3 Diethylaminopropylamin oder 2-Diethylaminoethylamin) zugetropft. Die Reaktionslösung wird 17 h bei Umgebungstemperatur gerührt, danach wird das Lösemittel abgezogen.

Die Überprüfung des Reaktionsansatzes mittels NMR ergab eine nahezu 100%ige Umsetzung.

In einem weiteren Ansatz wird ein Diamin (3-Dimethylaminopropylamin (DMAPA), 3-Diethylaminopropylamin (DEAPA) oder 2-Diethylaminoethylamin (DEAEA)) zusammen mit CDI in wasserfreiem THF gelöst. Das Reaktionsprodukt wird analog zu 1.1. aufgearbeitet.

### 1.2. Synthese von modifizierten Polyvinylakoholen (PVA)

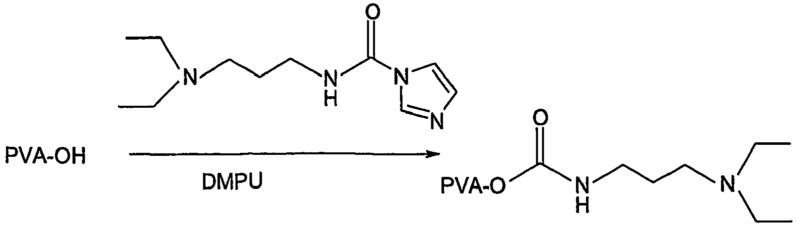

Eine gemäß Tabelle 1 angegebene Menge an PVA (Polymerisationsgrad (PG) 300) wird in 170 ml wasserfreiem NMP (N-Methylenpyrolidon) unter Stickstoffatmosphäre gelöst. Anschließend wird 1,3-Dimethyl-3,4,5,6-tetrahydro-2(H1)-pyrimidinon (DMPU) zugesetzt. Danach erfolgt die Zugabe des unter 1.1. hergestellten und in wasserfreiem NMP aufgenommenen Amin-Carbonylimidazols (Amin-Cl). Die Reaktionslösung wird bei 80 °C 4 bis 6 Tage gerührt. Anschließend wird der Ansatz ultrafiltriert und das erhaltene Polymer lyophilisiert und im Vakuum bei 43 °C zur Weiterverarbeitung gelagert

Eine qualitative Überprüfung des Reaktionserfolges wird mittels FT-IR (Nicolet FT-IR 510P) durchgeführt. Dazu wird das Polymer mit KBr in Pellet-Form gepresst. Der Nachweis der Substitution des Polyvinylakoholrückgrats erfolgt anhand des Signals der Urethangruppe bei 1696 cm⁻¹.

Zur Bestimmung des Anteils an Aminogruppen tragenden -Seitengruppen werden die erhaltenen Polymere in d₆-DMSO gelöst und mittels ¹H-NMR (Joel Eclipse 500; GX4000D) vermessen. Zur quantitativen Auswertung wurden die Signale der Amingruppe bei δ = 2,99, 2,21, 2,12, 1,53 (DMAPA) δ = 6,89; 2,99; 2,42; 2,36; 0,94 (DEAPA) herangezogen.

Die Ansätze und Ergebnisse sind in Tabelle 1 und 2 wiedergegeben.

**Tabelle 1:**

| | | |
|---|---|---|
| Eingesetztes Amin: Dimethylaminopropyamin (DMAPA) | | |

| | **Ansatz** | **Prozentualer Anteil an Aminsubstituenten** |
|---|---|---|
| **1** | 12,00g PVAL, 1,20g Amin-Cl, 0,07gDMPU, 200ml NMP | 2,3% |
| **2** | 12,00g PVAL, 2,40g Amin-Cl, 0,16g DMPU, 200ml NMP | 4,4% |
| **3** | 12,00g PVAL, 4,80g Amin-Cl, 0,31 g DMPU, 170ml NMP | 7,1% |
| **4** | 12,00g PVAL, 11,99g Amin-Cl, 0,78g DMPU, 170ml NMP | 10,8% |
| **5** | 11,00g PVAL, 23,08g Amin-Cl, 1,51g DMPU, 170ml NMP | 23,0% |
| Eingesetztes Amin: Diethylaminopropylamin: (DEAPA) | | |

| | **Ansatz** | **Prozentualer Anteil an Aminsubstituenten** |
|---|---|---|
| **6** | 10,00g PVAL, 1,00g Amin-Cl, 0,07gDMPU, 200ml NMP | 2,1% |
| **7** | 10,00g PVAL, 2,01g Amin-Cl, 0,06g DMPU, 170ml NMP | 4,0% |
| **8** | 10,00g PVAL, 4,02g Amin-Cl, 0,11 g DMPU, 170ml NMP | 5,9% |
| **9** | 10,00g PVAL, 10,05g Amin-Cl, 0,23g DMPU, 120ml NMP | 10,9% |
| **10** | 10,00g PVAL, 23,12g Amin-Cl, 1,32g DMPU, 170ml NMP | 22,7% |

Zur Überprüfung der durch die NMR gewonnenen Ergebnisse werden 5 mg des erhaltenen Polymers mit einem TGA7 (Perkin Elmer) unter Stickstoffatmosphäre bei 20 °C im Vakuum thermo-gravimetrisch untersucht. Die Ergebnisse sind in Tabelle 2 zusammengefasst.

**Tabelle 2:**

| **Polymer (PG 300)** | **Molekulargewicht (aus ¹H-NMR)** | **Anzahl der Amingruppen tragenden Seitenketten pro Polymer** | **Massenverlust bestimmt mit TGA nach dem ersten Abbauschritt (250-400°C)** | **Massenverlust erwartet nach ¹H-NMR** |
|---|---|---|---|---|
| **1** | 15318,14 | 2,25 % | 4,81 % | 7,59 % |
| **2** | 15885,98 | 4,35 % | 11,80 % | 14,15 % |
| **3** | 16754,20 | 7,10 % | 20,04 % | 21,89 % |
| **4** | 17875,22 | 10,76 % | 36,94 % | 31,10 % |
| **5** | 22424,22 | 23,04 % | 60,67 % | 53,08 % |
| **6** | 15461,20 | 2,10 | 9,02 | 8,16 |
| **7** | 16100,74 | 4,00 | 12,81 | 14,93 |
| **8** | 16692,34 | 5,90 | 38,19 | 21,24 |
| **9** | 18406,27 | 10,87 | 40,53 | 35,48 |
| **10** | 2311,77 | 22,70 | 58,47 | 57,04 |

### 1.3. Graft-Polymerisation mit L-Lacitd

0,500 g des modifizierten PVA-Rückgrats aus 1.2. werden mit 5,00 g L-Lactid in einem Rundkolben unter Stickstoffatmosphäre umgesetzt. Zu dem Reaktionsansatz werden 0,113 g SnOct₂ als Katalysator addiert. Zur Initiierung der Polymerisation wird der Kolben in ein vorgewärmtes 150 °C heißes Ölbad gesenkt, wobei die Reaktionslösung unter Verwendung eines Magnetrührers kontinuierlich gerührt wird. Nach 3 h wird das Ölbad durch ein kaltes Wasserbad ersetzt, danach wird zu der Reaktionsmischung Aceton aufgenommen. Polymerlösung wird in 250 ml Wasser/Isopropanol (1:1) präzipitiert. Nach Filtration des Reaktionsproduktes wird das erhaltene Polymer bei Umgebungstemperatur im Vakuum getrocknet. Der Erfolg der Umsetzung kann mittels ¹H-NMR überprüft werden.

### 1.4. Herstellung von Poly(laktid-co-glykolid) Gruppen tragenden Polyvinylalkoholen

Das erhaltene Amin-modifizierte Polymer wird mit einer Mischung aus D,L-Laktid und Glykolid (1:1) umgesetzt. Das Verhältnis des eingesetzten Polymers zu der Laktid/Glykolid-Mischung beträgt in den durchgeführten Ansätzen 1:1; 1:2; 1:10 und 1:20 stöchiometrische Anteile bezogen auf die freien Hydroxygruppen des PVA-Rückgrats. Der Reaktionsansatz wird mit ca. 10 mol% Sn(II) 2-ethylhexanoat und SnOct₂ versetzt. Nach einer Polymerisationsdauer von 3 h bei 150 °C wird der Reaktionsansatz schnell auf Umgebungstemperatur abgekühlt. Die Reaktionsmischung wird in Aceton aufgenommen und zur Reinigung mit einer Mischung aus Isopropanol/Wasser oder Wasser präzipitiert. Das isolierte Polymer wird bei 20 °C im Vakuum getrocknet.

Der Erfolg der Umsetzung wird mittels ¹H-NMR überprüft, das Ergebnis ist beispielhaft für Polymer Ansatz **10** aus Beispiel 1.2 und PVA :Glykolid/Lactid Ansatz von 1:20 wiedergegeben.
¹H-NMR: δ = 5,56-5,03, 2.04-1,54 (PVAL-Signale)
5,21 (Lactid), 4,22, 1,46 (Lactid-Endgruppe),
1,30 (Lactiden-Endgruppe)
4,86 (Glycolid), 4,08 (Glykolid-Endgruppe)
Integrale: Lactid Zentral zu End 11:1
Integrale: Glycolid zu Lactid Endgruppen 1,6 : 21,8 das ergibt ein Verhältnis von Lactid : Glycolid in Seitenketten: 56 : 44.

### Beispiel 2: Präparation von kolloidalen Partikeln

10 mg des Kammpolymers (ca. 8% Diethylaminopropylamin-Seitenketten (DEAPA); PVA zu Laktid/Glykolid 1: 20) hergestellt gemäß Beispiel 1. werden in ca. 3 ml Aceton gelöst. In 10 ml Reinstwasser werden 100 µl Tris-EDTA Puffer (low ionic strength 100x), eine definierte Menge an Stabilisator (als polymere Stabilisatoren werden verwendet: Carboxymethylcellulose (CMC); Tylopur ® C 600, Hoechst AG; als niedermolekularer Stabilisator wird Rose Bengal verwendet) und 0,5 g Glucose gegeben. Der pH-Wert der wässrigen Lösung wird mit HCl/NaOH auf 7,0 eingestellt. Die wässrige Lösung wird anschließend mit einem Nitrocellulose-Membranfilter mit einer Porengröße von 0,22 µm steril abfiltriert. Die polymerhaltige Lösung wird durch eine 20Gx1½ Kanüle einer 5 ml Spritze langsam, mit einer Geschwindigkeit von ca. 0,3 ml/min, in eine wässrige Phase, die in einem Becherglas mit einem Magnetrührer vorgelegt ist, gespritzt. Während des Einspritzens wird die Kanülenöffnung leicht an die Wand des Becherglases gedrückt. Die resultierende Suspension wird 4 h unter leicht reduziertem Druck gerührt bis das Aceton entfernt ist.

Die so hergestellten kolloidalen Lösungen können ohne Veränderung der kolloidalen Partikel zerstäubt werden.

Der Einfluss des negativ geladenen Stabilisators auf die Bildung und die Eigenschaften von kolloidalen Partikeln sind in den Tabellen 3 und 4 dargestellt. Die Kolloidbildung wird optisch beobachtet, wobei eine opaleszente Mischung die Bildung kolloidaler Partikel anzeigt.

**Tabelle 3:**

| **Stabilisator** | **Menge (µg)** | **Partikeldurch messer (nm)** | **Zeta-Potential (mV)** | **Beobachtung** |
|---|---|---|---|---|
| CMC | 2000 | 213,6 ±3.0 | -46,6 ±1,1 | opaleszent |
| | 1000 | 215,5 ±2,9 | -28,4 ±0,4 | opaleszent |
| | 500 | 202,5 ±1,5 | -23,0 ±0,5 | opaleszent |
| | 250 | 202,5 ±2,9 | -16,6 ±0,3 | wolkig trüb |
| | 100 | 256,6 ±4.6 | 39,9 ±0,3 | wolkig/flockig |
| | 50 | 241,4 ±1,9 | 45,6 ±0,7 | wolkig/flockig |
| | 25 | 177,8 ±1,0 | 47,6 ±1,7 | opaleszent |
| | 0 | 76,2 ±8,8 | 58,9 ±1,9 | klar |

**Tabelle 4 (nicht gemäß der Erfindung):**

| **Stabilisator** | **Menge (µg)** | **Menge (µmol)** | **Partikeldurch messer (nm)** | **Zeta Potential (mV)** | **Beobachtung** |
|---|---|---|---|---|---|
| Rose Bengal | 2840 | 0,26 | 392,8 ±7,9 | --- | wolkig |
| | 1360 | 0,17 | 246,7 ±6,1 | -22,2 ±0,4 | opaleszent |
| | 900 | 0,11 | --- | --- | flockig |

Die Bestimmung der Größe der kolloidalen Partikel wird mittels Photonen-Korrelations- Spektrometrie (PCS) durchgeführt. Die Messung des Zeta-Potentials erfolgt mittels Laser-Doppler-Anemometrie mit Hilfe eines Zetasizer 4/AZ 104 (Malvern Instruments, Malvern, UK).

### Beispiel 3: Prüfung der Vernebelungsstabilität

Es wird eine Suspension aus CMC-haltiger kolloidaler Partikel gemäß Beispiel 2 hergestellt.

Analog wird eine Suspension kolloidaler RG 503 Partikel (50 mg in 10 ml Aceton; Polymer RG 503 Resomer ®, Boehringer Ingelheim, Deutschland) mit 0,0025% Alveofact ® (Boehringer Ingelheim) mit der Solvent-Displacement-Methode (Jung T., et al. J. Controlled Release 67, 157-169, (2000)) erzeugt.

Die Suspensionen werden mit einem kommerziellen Düsenvernebeler (Pari LC Star) unter anlegen eines Druckluft-Gasflusses durch den Vernebeler von 20 l/min zerstäubt. Das Aerosol wird mit einer gereinigten Glasplatte aufgefangen. 1 ml des aufgefangenen Aerosols werden anschließend in 29 ml destilliertem Wasser aufgenommen. Zur Charakterisierung des vernebelten Aerosols werden die aufgenommenen Suspensionen CMC haltiger kolloidaler Partikel und als Vergleich der kolloidalen RG 503 Partikel mit einem Laser-Diffraktometer (Sympatec GmbH, Claustal-Zellerfeld, Deutschland) vermessen. Die Ergebnisse sind in Fig. 1 dargestellt.

Bei der Interpretation der Messdaten, erhalten durch die Laser-Diffraktion ist zu beachten, dass die verwendete Messmethode sehr gut geeignet ist Veränderungen in der Partikelgrößenverteilung zu bestimmen. Die absoluten Werte für den mittleren Durchmesser kleiner Partikel (kleiner 1 µm) können wesentlich genauer mittels Photonen-Korrelations- Spektroskopie (PCS) bestimmt werden. Die entsprechenden Werte sind in Beispiel 2, Tabelle 3 angegeben.

Wie in Fig. 1 gezeigt, zeichnen sich die erfindungsgemäßen Partikel durch eine ungewöhnlich gute Vernebelungsstabilität aus. Die Partikelgrößeverteilung vor (Fig. 1 a)) und nach (Fig. 1 b)) der Vernebelung bleibt nahezu erhalten. RG 503 besitzt im unvernebelten Zustand (Fig. 1 c) eine ähnliche Partikelgrößenverteilung wie die beanspruchten Partikel. Nach der Vernebelung besitzt RG 503 allerdings eine starke Neigung zur Aggregation (Fig. 1 d), wodurch der Anteil von Partikeln mit größeren Durchmesser stark ansteigt.

## Patentansprüche

1. Bioabbaubare kolloidale Partikel umfassend
a) amphiphile Kammpolymere enthaltend ein wasserlösliches Polyol-Rückgrat mit einem Polymerisationsgrad von 300 hydrophobe Seitenketten und sekundäre, tertiäre oder quartäre Aminogruppen tragende Seitenketten und
b) als Stabilisator Carboxymethylcellulose (CMC) und einen oder mehrere pharmazeutische Wirkstoffe, wobei mindestens ein Wirkstoff eine negativ geladene Bse oder deren Korrespondierende säure ist, wobei
■ die sauren Gruppen der Stabilisatoren im Über- oder Unterschuss gegenüber den sekundären oder tertiären Aminogruppen der Kammpolymere vorliegen, oder die basischen Gruppen im Unter- oder Überschuss gegenüber den quartären Aminogruppen der Kammpolymeren vorliegen, so dass die kolloidalen Partikel ein positives oder negatives Zeta-Potential aufweisenund
■ das Kammpolymer hydrophobe Seitenketten aus der Gruppe der Poly(laktid-co-glycolide) besitzt, und
■ die Aminogruppen tragenden Seitenketten die Strukturformel 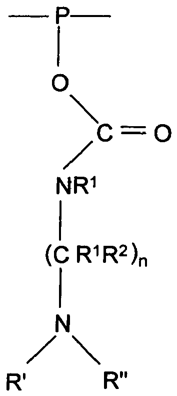
besitzen, für die gilt:
n=3,
P steht für Polyolrückgrat, welches aus der Gruppe der Polyvinylalkohole ausgewählt wurde,
R', R" sind C₂-Alkylgruppen,
R¹ = R² = H,
wobei die Aminogruppen tragenden Seitenketten in einem Anteil von 8% vorhanden sind,
wobei das Gewichtsverhältnis des Stabilisators Carboxymethylcellulose zum Kammpolymer 0,5 bis 2 : 10 beträgt,
wobei das Verhältnis von Laktid zu Glykolid in den hydrophoben Seitenketten 56 : 44 beträgt, und
wobei das Verhältnis des Polyvinylalcohols zu Laktid/Glykolid 1 : 20 beträgt.

2. Verwendung von bioabbaubaren kolloidalen Partikeln gemäß Anspruch 1 zur Herstellung eines Medikaments zur Behandlung von pulmonalen und systemischen Erkrankungen beim Menschen und beim Tier.

3. Verwendung von bioabbaubaren kolloidalen Partikeln gemäß Anspruch 1, **dadurch gekennzeichnet, dass** das Medikament ein zur inhalativen (pulmonalen) Applikationgeeignetes Medikament ist.

4. Verwendung gemäß einem der Ansprüche 2 bis 3, **dadurch gekennzeichnet, dass** die bioabbaubaren kolloidalen Partikeln in einer physiologisch verträglichen wässrigen Lösung vorliegen.

5. Verfahren zur Herstellung von bioabbaubaren kolloidalen Partikeln gemäß Anspruch 1 umfassend folgende Verfahrensschritte
a) lösen eines Kammpolymers, enthaltend ein wasserlösliches Polyol-Rückgrat, hydrophoben Seitenketten und sekundäre, tertiäre oder quartäre Aminogruppen tragende Seitenketten in Aceton,
b) versetzen einer isotonischen wässrigen Lösung mit einem pH-Wert von 7,0 enthaltend neben einem Zucker, namentlich Glucose, und einem Puffer, namentlich Tris-EDTA CMC mit der Lösung erhalten aus a),
c) rühren der aus b) erhaltenen Lösung zur Herstellung kolloidaler Partikel und
d) entfernen des Acetons.

## Claims

1. Biodegradable colloidal particle comprising
a) amphiphilic comb polymers comprising a water-soluble polyol backbone with a degree of polymerization of 300, hydrophobic side chains and side chains carrying secondary, tertiary or quaternary amino groups and
b) carboxymethyl cellulose (CMC) as a stabilizer and one or more pharmaceutical agents, at least one agent being a negatively charged base or its corresponding acid,
• the acid groups of the stabilizers being available in excess or deficiency compared to the secondary or tertiary amino groups of the comb polymers or the basic groups being available in excess or deficiency compared to the quaternary amino groups of the comb polymers, so that the colloidal particles comprise a positive or negative zeta potential and
• the comb polymer having hydrophobic side chains from the group of the poly(lactide-co-glycolides), and
• the side chains carrying amino groups having the structural formula 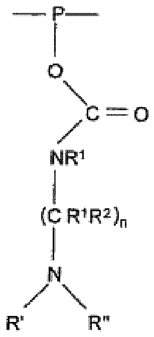 to which the following applies:
n=3,
P stands for polyol backbone, which was selected from the group of the polyvinyl alcohols,
R', R" are C₂ alkyl groups
R¹ = R² = H,
the side chains carrying amino groups being present in a percentage of 8%,
the weight ratio of the stabilizer carboxymethyl cellulose to the comb polymer being 0.5 to 2 : 10,
the ratio of lactide to glycolide being 56 : 44 in the hydrophobic side chains, and
the ratio of the polyvinyl alcohol to lactide/glycolide being 1 : 20.

2. Use of biodegradable colloidal particles according to claim 1 for the production of a pharmaceutical for the treatment of pulmonary and systemic diseases in humans and animals.

3. Use of biodegradable colloidal particles according to claim 1, **characterized in that** the pharmaceutical is a pharmaceutical appropriate to inhalative (pulmonary) application.

4. Use according to one of the claims 2 to 3, **characterized in that** the biodegradable colloidal particles are available in a physiologically compatible aqueous solution.

5. Method for the production of biodegradable colloidal particles according to claim 1 comprising the following processing steps
a) dissolving a comb polymer comprising a water-soluble polyol backbone, hydrophobic side chains and side chains carrying secondary, tertiary or quaternary amino groups in acetone,
b) adding a solution obtained from a) to an isotonic aqueous solution with a pH value of 7.0 comprising CMC in addition to a sugar, namely glucose, and a buffer, namely tris-EDTA,
c) stirring the solution obtained from b) for the production of colloidal particles and
d) removing the acetone.

## Revendications

1. Particules colloïdales biodégradables comprenant
a) des polymères en peigne amphiphiles contenant une chaîne principale de polyol hydrosoluble avec un degré de polymérisation de 300, des chaînes latérales hydrophobes et des chaînes latérales portant des groupes amino secondaires, tertiaires ou quaternaires et
b) carboxyméthylcellulose (CMC) comme stabilisateur et un ou plusieurs principes actifs pharmaceutiques, où au moins un principe actif est une base chargée négativement ou son acide correspondant, où
• les groupes acides des stabilisateurs sont en excès ou en défaut par rapport aux groupes amino secondaires ou tertiaires des polymères en peigne, ou les groupes de base sont en excès ou en défaut par rapport aux groupes amino quaternaires des polymères en peigne, de sorte que les particules colloïdales présentent un potentiel zêta positif ou négatif zêta et
• le polymère en peigne possède des chaînes latérales hydrophobes du groupe des poly(lactide-co-glycolides), et
• les chaînes latérales portant les groupes amino possèdent la formule: 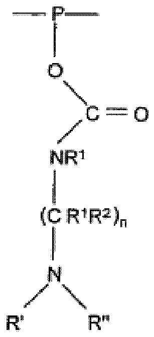 pour laquelle:
n=3,
P représente la structure polyol qui est choisie parmi le groupe des alcools polyvinyliques,
R', R" sont des groupes alkyles en C₂
R¹=R²=H ,
où les chaînes latérales portant des groupes amino sont présents à hauteur de 8%,
où le rapport pondéral du stabilisateur carboxyméthylcellulose par rapport au polymère en peigne est de 0,5 à 2 : 10,
où le rapport du lactide au glycolide dans les chaînes latérales hydrophobes est de 56 : 44, et
où le rapport de l'alcool polyvinylique au lactide/glycolide est de 1 : 20.

2. Utilisation de particules colloïdales biodégradables selon la revendication 1 pour la fabrication d'un médicament pour le traitement des maladies pulmonaires et systémiques chez l'homme et les animaux.

3. Utilisation de particules colloïdales biodégradables selon la revendication 1, **caractérisée en ce que** le médicament est un médicament approprié pour une application inhalatrice (pulmonaire).

4. Utilisation selon l'une des revendications 2 à 3, **caractérisée en ce que** les particules colloïdales biodégradables sont présentes dans une solution aqueuse physiologiquement compatible.

5. Procédé de fabrication de particules colloïdales biodégradables selon la revendication 1, comprenant les étapes suivantes du procédé
a) dissoudre dans de l'acétone un polymère en peigne contenant une chaîne principale de polyol hydrosoluble, des chaînes latérales hydrophobes et des chaînes latérales portant des groupes amino secondaires, tertiaires ou quaternaires,
b) ajouter la solution obtenue en a) à une solution isotonique aqueuse à pH de 7.0 contenant le CMC en plus d'un sucre, à savoir du glucose, et un tampon, à savoir le tris-EDTA,
c) agiter la solution obtenue par b) pour la production de particules colloïdales et
d) enlever l'acétone.
